# EUROPEAN PATENT SPECIFICATION

(11) **EP 0 610 418 B1**
(45) Date of publication and mention of the grant of the patent: **18.08.1999**
(21) Application number: 92924046.3
(22) Date of filing: 28.10.1992
(51) Int. Cl.: G01F 3/00, A61M 5/168

(54) **METHOD FOR LIQUID FLOW MEASURING AND APPARATUS TO PRACTISE THIS METHOD**
VERFAHREN UND VORRICHTUNG ZUR MESSUNG DER FLÜSSIGKEITSSTRÖMUNG
PROCEDE DE MEUSRE DU DEBIT D'UN LIQUIDE, ET APPAREIL POUR SA MISE EN OEUVRE

(30) Priority: 30.10.1991 NL 9101825
(43) Date of publication of application: 17.08.1994
(73) Proprietor: ROELOFS OCTROOIEN EN INVESTERINGEN B.V., 5628 NM Eindhoven (NL)
(72) Inventor: ROELOFS, Bernardus, Johannes, Gerardus, Maria, NL-5628 NM Eindhoven (NL)
(74) Representative: Scheele, Edial François
(86) International application number: PCT/NL92/00193
(87) International publication number: WO 93/09407

(56) References cited:
- FR-A- 2 458 804
- GB-A- 2 207 239
- US-A- 3 609 379
- US-A- 4 328 801
- US-A- 4 936 828
- PROCEEDINGS COMPUTERS IN CARDIOLOGY , PUBL. 1990, LOS ALAMITOS, CALIFORNIA, US pages 405 - 406 R. LEOR, E.A. 'A SYSTEM FOR THE MEASUREMENT OF DROP VOLUME OF INTRAVENOUS SOLUTIONS'

## Description

The invention pertains to a method of measuring the flow rate of a liquid passing through an outlet using optical imaging and photoelectric detection means and to an apparatus to carry out this method.

Such a method and apparatus are known from US 4,936,828. Therein a camera is used to measure the volume of falling drops and the number of falling drops per unit of time is counted in order to calculate the flow of the liquid. The smallest measuring volume unit is thus the volume of a complete falling drop. Major drawbacks of this known method and equipment are: its inaccuracy, its noise sensitivity and the influence of evaporation, vibration and viscosity of the liquid on the measuring. The method as disclosed is thus less useful for measurement of especially small flow rates such as for e.g. medical infusion systems where often very small quantities are involved.

It is an object of the invention to provide a method and apparatus working accurate, real time and being particularly useful for especially relatively small flow rates.

A method as introduced in the first paragraph above is according to the invention characterized in that said means continuously measure volume growth of a droplet of liquid during building up at the liquid outlet, said volume growth defining directly the flow rate of said liquid. The apparatus of the invention is defined in claim 4.

Measuring he growth of droplets during growth with the use of optical imaging and image processing means enables the determination of volume flow rate in real time with a high accuracy and results in a flexible measuring set-up to take into account other relevant parameters like environmental back-pressure.

For optical registration use is made preferably of a detector matrix like a CCD chip, a conventional video camera, an open RAM memory or other light sensitive devices. Such a detector is arranged along the location of the growing drop.

Although it may be supposed that the shape of the drop hanging at a cylindrical, vertical, outlet has due to gravity a cylindrical symmetry, in a preferred embodiment use is made of image acquisition in more directions through a growing drop. In this situation more acquisition devices can be applied in a known manner, but also an image from different directions can be projected on the detector using optical tools, like mirrors. In such an arrangement corrections can be made for asymmetry and/or vibrations in the drop. Hence the accuracy of the measuring method can be improved and the consequences of the environmental conditions minimized.

Determination of the volume increment of the drops in a unit of time is used in a preferred embodiment for flow rate measuring and for controlling and adjusting any flow rate as long as drop formation occurs.

In a further embodiment use is made of liquid flow rate measuring data to control the liquid flow in a feedback loop. The increase of the volume of liquid in a unit of time can be used for flow rate measuring and for controlling and adjusting any flow rate.

With such automated measuring methods a feed-back controlled and adjusted infusion pump can be realised. Also existing pumps can be measured and checked for all the relevant parameters.

This measuring method can be applied in a wide range of instruments applicable for dosing, measuring or controlling and regulating.

Two applications can be distinguished for measuring and dosing purposes. The measuring of the flow rates of pumps to be checked can be executed by measurements on drops as well as measurements on a container. A pump for a certain, continuous adaptable, flow rate can be designed using the drop measurement for feed-back of the real time yield of the flow to adjust the speed of the pump and hence that certain flow rate.

A container for the collected liquid has the shape of a transparent cylinder. Once filled, this cylinder acts as a cylinder lens and hence the level of the meniscus can be determined appropriate due to the difference in optical properties between the filled and empty part of the container. Thereto an illumination slit is preferably positioned parallel to the axis of the cylinder opposite to the image acquisition device in the back focal plane of the cylinder lens formed by the filled container. In case of an empty container the container acts as a window so the slit will not be magnified. This changes in the case of a full container where the slit will be magnified onto the pick-up device. Both parameters thus can be used for a real time dosing equipment.

A method as disclosed is particularly applicable in an apparatus for an accurate determination of liquid flow rates such as for example in medical infusion apparatus.

In a preferred embodiment an apparatus according to the invention is provided with a data processing unit for checking the flow rate and/or the yield of a liquid dosing system upon the flow rate data obtained. Such an apparatus is in particular applicable to check the yield for dosing in medical treatment or diagnosis.

In a further embodiment an apparatus according to the invention is provided with a data processing unit to adjust a liquid dosing system upon flow rate data obtained. Such an apparatus is in particular applicable to control and/or adjust a flow of a dosing system of e.g. a medical infusion set.

The invention will be described in more detail with reference to the drawing in which:
Figure 1 shows an overview of a set-up, according to the invention, and an optical imaging part thereof;
Figure 2 shows an example of a flow-feed-back regulated pump.

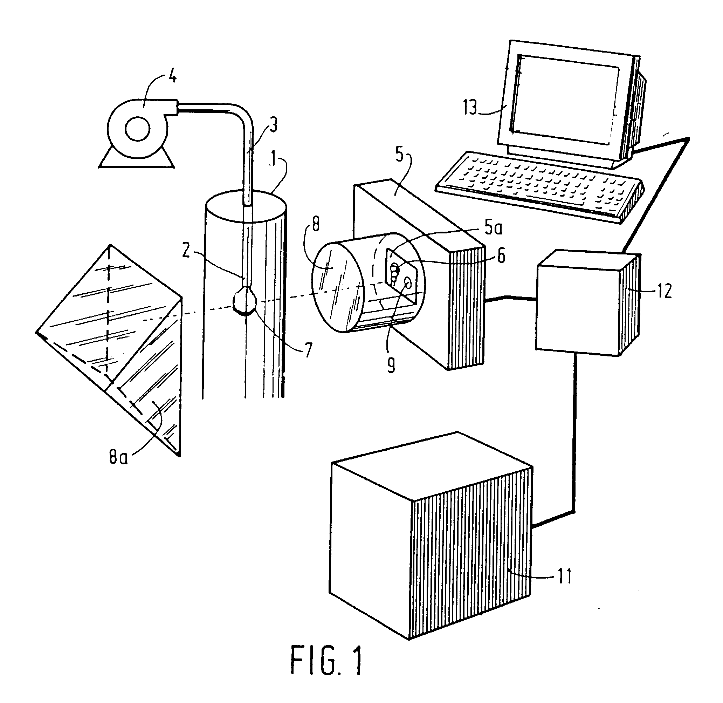

A measuring set-up as represented in Figure 1 shows schematically a support 1 with, closed to an outlet 2 of a tubing 3 of a pump 4 to be checked, an imaging device 5 with a pick-up entrance screen 5a on which an image 6 of a growing drop 7 at the outlet is projected by a lens system 8. Here a prism 8a projects an image 9 of a falling drop 10 also on the pick-up device 5. Similar set-ups 11 can be positioned under an angle with the described set-up for minimizing the influence of asymmetries. Images registered by the pick-up device 5 are processed by an image processor 12 which calculates the drop growth and represents the value or reprocessed results on a presentation peripheral 13.

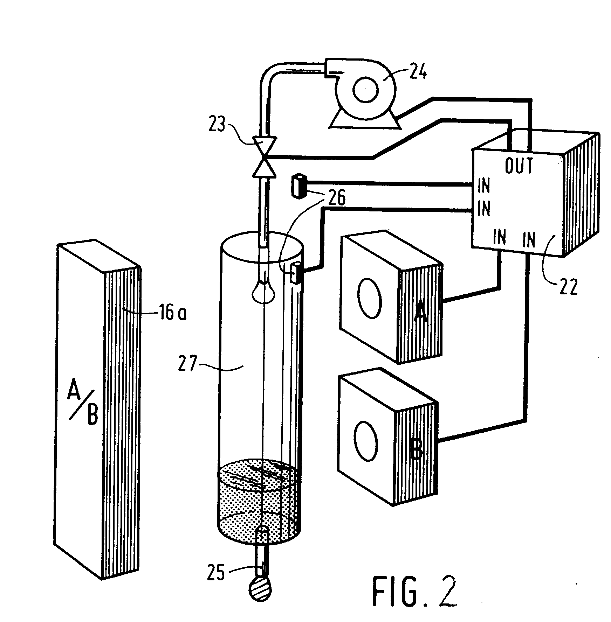

In Figure 2 a set-up A for drop measurement and a set-up B for meniscus detection, are combined for regulation of a flow itself. Set-up A regulates a driving force needed for meeting a required flow. An illuminating device 16a is part of the set-up devices A and B. A processor unit 22 steers a valve 23 or the speed of a pump 24. Set-up B detects a meniscus level to let compensate a back pressure at an outlet 25 of the complete device. Pressure sensors 26 in the outer world and a drop chamber 27 can also support the control strategy due to their even faster sampling speed. A combination of set-up A and set-up B presents the principles of a general dosing system which can be applied e.g. profitably in infusion systems for medical 5 use. Due to the technique of drip chambers, which are in use already for a very long time in hospitals, the method is easily acceptable.

## Claims

1. Method of measuring the flow rate of a liquid passing through an outlet using optical imaging and photoelectric detection means characterized in that said means continuously measure volume growth of a droplet of liquid during building up at the liquid outlet, said volume growth defining directly the flow rate of said liquid.

2. Method as claimed in Claim 1 wherein drop volume growth is performed along more directions in a plane square to the falling path of the drops.

3. Method as claimed in Claim 1 or 2 wherein liquid flow rate measuring data are used to control the liquid flow rate in a feedback loop.

4. Apparatus arranged to carry out the method as claimed in Claim 1, 2 or 3 wherein a measured flow rate is used to control and/or adjust a dosing system e.g. a medical infusion set, said apparatus being provided with a drop outlet and optical imaging and photoelectic detection means arranged to measure volume growth of a droplet during building up at the outlet.

5. Apparatus as claimed in claim 4, said apparatus being further provided with a transparent container to collect the liquid dropping from said outlet and with further optical imaging and photoelectric detection means to determine the change in the location of a meniscus of said liquid in the container to deliver a further signal for said control.

## Patentansprüche

1. Verfahren zur Messung der Flüssigkeitsströmung einer durch einen Auslass strömenden Flüssigkeit, wobei optische Bild- und photo-elektrische Detektionsmittel eingesetzt werden, dadurch gekennzeichnet, dass diese Mittel ständig den Volumenzuwachs eines sich an dem Flüssigkeitsauslass bildenden Flüssigkeitstropfens messen, welcher Volumenzuwachs direkt die Strömungsgeschwindigkeit der Flüssigkeit bestimmt.

2. Verfahren nach Anspruch 1, wobei der Zuwachs des Tropfenvolumens in mehreren Richtungen in einer Ebene quer zur Fallrichtung der Tropfen erfolgt.

3. Verfahren nach Anspruch 1 oder 2, wobei die Messdaten der Flüssigkeitsströmungsgeschwindigkeit dazu benutzt werden, in einer Rückkopplungsschleife die Flüssigkeitsströmungsgeschwindigkeit zu regeln.

4. Vorrichtung, geeignet zur Durchführung des Verfahrens nach Anspruch 1, 2 oder 3, die eine gemessene Strömungsgeschwindigkeit für die Regelung und/oder Einstellung eines Dosiersystems verwendet, zum Beispiel eines medizinischen Infusionsgeräts, welche Vorrichtung mit einem Tropfenauslass und Bild- und photo-elektrischen Detektionsmitteln zum Messen des Volumenzuwachses eines sich am Auslass bildenden Tropfens versehen ist.

5. Vorrichtung nach Anspruch 4, wobei die Vorrichtung weiters eine durchsichtiges Behältnis zum Auffangen der aus dem Auslass heraustropfenden Flüssigkeit sowie ergänzende optische Bild- und photo-elektrische Detektionsmittel aufweist zur Feststellung der Ortsveränderung eines Meniskus der Flüssigkeit in dem Behältnis, um ein ergänzendes Signal für die Regelung zu bieten.

## Revendications

1. Procédé pour le mesurage de la vitesse du courant d'un liquide coulant par un échappement, en utilisant des moyens de détection d'images et photo-électriques optiques, caractérisé en ce que ces moyens mesurent continuellement l'accroissement du volume d'une goutte de liquide se formant à l'échappement du liquide, lequel accroissement du volume détermine directement la vitesse du courant du liquide.

2. Procédé selon revendication 1, l'accroissement du volume de la goutte ayant lieu dans plusieurs directions sur un plan en travers du sens de la chute des gouttes.

3. Procédé selon revendication 1 ou 2, les données du mesurage de la vitesse du courant du liquide étant utilisées afin de régler la vitesse du courant du liquide dans une boucle de rétroaction.

4. Dispositif approprié à l'exécution du procédé selon revendication 1, 2 ou 3, en utilisant une vitesse du courant mesurée pour régler et/ou mettre au point un système de dosage, par exemple un appareil médical de perfusion, lequel dispositif est pourvu d'un échappement des gouttes et de moyens de détection d'images et photo-électriques pour le mesurage de l'accroissement du volume d'une goutte se formant à l'échappement.

5. Dispositif selon revendication 4, le dispositif contenant en outre une coupe transparente pour recevoir le liquide dégouttant de l'échappement et des moyens de détection d'images et photo-électriques optiques supplémentaires afin de constater le déplacement d'un ménisque du liquide dans la coupe, afin de pourvoir un signal supplémentaire de réglage.
